**Europäisches Patentamt**

(19) **European Patent Office**

**Office européen des brevets**

(11) Veröffentlichungsnummer: **0 220 379**
**B1**

(12) **EUROPÄISCHE PATENTSCHRIFT**

(45) Veröffentlichungstag der Patentschrift:
09.05.90

(51) Int. Cl.5: **C12N 5/02**

(21) Anmeldenummer: **86109103.1**

(22) Anmeldetag: **03.07.86**

(54) Verwendung von Glutamin in Form seiner Di- und Tripeptide im Nährmedium für Zellkulturen.

(30) Priorität: **28.10.85 DE 3538310**

(43) Veröffentlichungstag der Anmeldung:
**06.05.87 Patentblatt 87/19**

(45) Bekanntmachung des Hinweises auf die Patenterteilung:
**09.05.90 Patentblatt 90/19**

(84) Benannte Vertragsstaaten:
**AT BE CH DE FR GB IT LI LU NL SE**

(56) Entgegenhaltungen:
**EP-A- 0 087 750**

**D.W. BARNES et al.: "METHODS FOR PREPARATION OF MEDIA, SUPPLEMENTS, AND SUBSTRATA FOR SERUM-FREE ANIMAL CELL CULTURE", 1984, Seite 41, Alan R. Liss, Inc., New York, US**
**JOURNAL OF CHROMATOGRAPHY, Band 294, 1984, Seiten 507-512, Elsevier Science Publishers B.V., Amsterdam, NL; P. STEHLE et al.: "Isotachophoretic analysis of a synthetic dipeptide L-alanyl-L-glutamine. Evidence for stability during heat sterilization")TR. 1973, 8(8), 293-5 000**

(73) Patentinhaber: **PFRIMMER KABI GMBH & CO. KG, Hofmannstrasse 26, D-8520 Erlangen(DE)**

(72) Erfinder: **Roth, Erich, Dr., Alser Strasse 4, A-1090 Wien IX(AT)**

(74) Vertreter: **Freiherr von Pechmann, Eckehart et al, Wuesthoff & Wuesthoff Patent- und Rechtsanwälte Schweigerstrasse 2, D-8000 München 90(DE)**

## Beschreibung

Die in vitro-Kultivierung von tierischen und pflanzlichen Zellen ist ein wichtiges Instrument in der biologischen Forschung geworden. So werden in zunehmenden Maße Zellkulturen meist als biologische Modelle verwendet, um z.B. pharmakologische Wirkungen neuer chemischer Substanzen zu testen, wodurch bisher notwendige Tierversuche bei der Untersuchung derartiger Substanzen zum Teil ersetzt werden können. Auch ist es möglich geworden, bestimmte Stoffe, z.B. monoklonale Antikörper in derartigen Zellkulturen im industriellen Maßstab herzustellen. Ferner werden auch Zellkulturen im großen Maße bei der Tumorforschung eingesetzt. Auch in Verbindung mit der extrakorporalen Befruchtung zur Überwindung der Sterilität wird die isolierte Eizelle in Nährlösungen mit den Spermien in Kontakt gebracht und dann das befruchtete Ei bis zum Stadium des Vier - oder Achtzell-Embryos 48 - 60 Stunden inkubiert bevor der Transfer in den Uterus erfolgt. vgl. Arch. Gynerol 231, 171-176 und 321 -323 (1982) sowie Dtsch. med. Wschr. Bd 109, 547 -552 (1984).

Bei der künstlichen Züchtung der Zellen tierischer Gewebe hat sich das Glutamin schon vor vielen Jahren als wertvoller Wachstumfaktor für die Zellentwicklung erwiesen, weshalb es in den Kulturmedien bis zu einer 20-fachen größeren Konzentration als andere Aminosäure zugesetzt wird; vgl. H. Eagle, Nature 1955, Seiten 501 - 504. Glutamin wurde inzwischen auch neben Glukose als die wichtigste Energiequelle für das Wachstum von isolierten Säugetierzellen und auch Krebszellen erkannt. vgl. H.R. Zielke et al, Federation proceedings, Bl. 43, Nr.1, Seiten 121 bis 125, 1984 bzw. L.J. Reitzer et al, Journ. Biol. Chem., Seiten 2669 bis 2676, 1979.

Die notwendigen Kulturmedien zur Züchtung von isolierten Zellen werden bereits industriell hergestellt und kommerziell verwertet. Allerdings ist die Herstellung glutaminhaltiger Kulturmedien vom technologischen und vertriebstechnischen Standpunkt aus gesehen sehr schwierig, da Glutamin eine relativ instabile Verbindung ist. Deshalb werden die Kulturmedien meist im wesentlichen glutaminfrei geliefert und die Anwender müssen dann das Glutamin unter erhöhtem Aufwand steril zusetzen, was nicht immer durchführbar ist, u.a. wegen des Kontaminationsrisikos.

Der Grund für die Instabilität des Glutamins ist seine Neigung zur intramolekularen Cyclisierung insbesondere bei der thermischen Sterilisierung der glutaminhaltigen Lösungen. Diese cyclische Produkt besitzt jedoch toxische Eigenschaften auch gegenüber isolierten Zellen.

Bei Infusionslösungen für die künstliche Ernährung von Patienten hat man bereits maskiertes Glutamin in Form des $\alpha$-N acylierten Glutamins bzw. Di- und Tri-peptide des Glutamins eingesetzt, da dieses acylierte Glutamin bzw. diese Peptide bei der Hitzesterilisierung der betreffenden Lösungen stabil bleiben, jedoch dann im Organismus z.B. durch die im Blut oder Serum vorhandenden Hydrolasen, aufgespalten werden. vgl. Europäische Patentschrift 87 750.

Gegen die Verwendung eines in dieser Weise maskierten Glutamins, sprach bisher die Tatsache, daß in einem Kulturmedium, das für die künstliche Züchtung von Gewebszellen verwendet wird, die betreffenden Substrate in einer von den Zellen unmittelbar metabolisierbaren Form vorliegen müssen, da die Auffassung besteht, daß anders als z.B. die Mikroorganismen, wie Bakterien, Schimmelpilze, die Säugetierzellen keine Exoenzyme an die Kulturlösungen abgeben, die dann die Spaltung derartig maskierter Verbindungen bewirken können. Daher enthalten die Nährlösungen für Zellkulturen anders als diejenigen für das Züchten von Mikroorganismen nur freie Aminosäuren, also keine Oligopeptide oder diese enthaltenden Abbauprodukte von Proteinen oder Disacchariden und dgl. aufzuspaltende Substrate.

Es hat sich nun überraschenderweise gezeigt, daß bestimmte Di-/Tripeptide des Glutamins doch von den Zellen, insbesondere von den Säugetierzellen, als Glutaminquelle verwertet werden können. Dies ist deshalb besonders überraschend, weil z.B. das an der $\alpha$-Aminosäuregruppe acylierte Glutamin (Acetylglutamin) von den Zellkulturen nicht oder nur sehr schlecht verwertet wird. Die niederen Peptide sind dagegen universell verwertbar, auch bei Zellen der verschiedensten Gewebe.

Erfindungsgemäß wird daher bei den Kulturmedien für das Wachstum von Säugetierzellen bzw. pflanzlichen Zellen das Glutamin in Form der bestimmten Di- und/oder Tripeptide, wie sie im Patentanspruch genannt sind, verwendet, wodurch nunmehr die wäßrigen Kulturmedien hinsichtlich der Aminosäuren- und Peptidkomponenten ohne Schwierigkeiten hitzesterilisierbar sind. Diese neuen Kulturmedien sind dann auch bei längerer Lagerung ausreichend stabil.

Die erfindungsgemäß verwendeten niederen Peptide des Glutamins zeigen - wie aus beigefügten Figuren mit den Diagrammen der Ergebnisse von Wachstumstests isolierter Säuretierzellen ersichtlich - ein sehr gutes Wachstum der Zellen, während mit dem glutaminfreuen Medium oder bei Verwendung des an der $\alpha$-Aminogruppe acetylierten Glutamins nur ein langsammes bzw. ein verspätet einsetzendes Wachstum der Zellen festzustellen ist.

Auch als Kulturmedien für die Entwicklung der Eizelle zum mehrzelleigen Embryo bei der extrakorporalen Befruchtung eignen sich die unter Verwendung der Glutaminpeptide hergestellten Nährlösungen besonders gut. Die Zellentwicklung und -teilung setzt bereits kurz nach der künstlichen Befruchtung der Eizelle ein, so daß dann der entstandene Zellhaufen schon bald in den Uterus implantiert werden kann.

Aber auch zur Entwicklung pflanzlicher Zellen bei der Kultur von pflanzlichen Protoplasten oder pflanzlicher Gewebe und spezieller Organe sowie bei Meristemkulturen für die vegetative Vermehrung können diese Kulturmedien vorteilhaft verwendet werden.

Nachfolgend wird eine für die Kulturen verschiedener Zellen geeignete Zusammensetzung eines erfindungsgemäßen Kulturmediums angegeben.

B e i s p i e l

| Bestandteil | mg/Liter | Bestandteil | mg/Liter |
|---|---|---|---|
| L-Arginin | 200,00 | Biotin | 0,20 |
| L-Asparagin $H_2O$ | 56,82 | D-Calciumpantothenat | 0,25 |
| L-Asparaginsäure | 20,00 | Cholinchlorid | 3,00 |
| L-Cystin $Na_2$ | 59,15 | Folsäure | 1,00 |
| L-Glutaminsäure | 20,00 | i-Inosit | 35,00 |
| L-Alanyl-glutamin | 500,0 | Nicotinamid | 1,00 |
| Glutathion | 1,00 | p-Aminobenzoesäure | 1,00 |
| Glycin | 10,00 | Pyridoxin HCl | 1,00 |
| L-Histidin | 15,00 | Riboflavin | 0,20 |
| L-hydroxyprolin | 20,00 | Thiamin HCl | 1,00 |
| L-Isoleucin | 50,00 | Vitamin B12 | 0,005 |
| L-Leucin | 50,00 | $Ca(NO_3)_2$ | 69,49 |
| L-Lysin HCl | 40,00 | KCl | 400,0 |
| L-Methionin | 15,00 | $MgSO_4$ $7H_2O$ | 100,0 |
| L-Phenylalanin | 15,00 | NaCl | 6000 |
| L-Prolin | 20,00 | $NaHCO_3$ | 2000 |
| L-Serin | 30,00 | $Na_2HPO_4$ | 800,7 |
| L-Threonin | 20,00 | Glucose | 2000 |
| L-Tryptophan | 5,00 | Phenolrot | 5,00 |
| L-Tyrosin | 20,00 | Aqua dest. | ad 1 Liter |
| L-Valin | 20,00 | | |

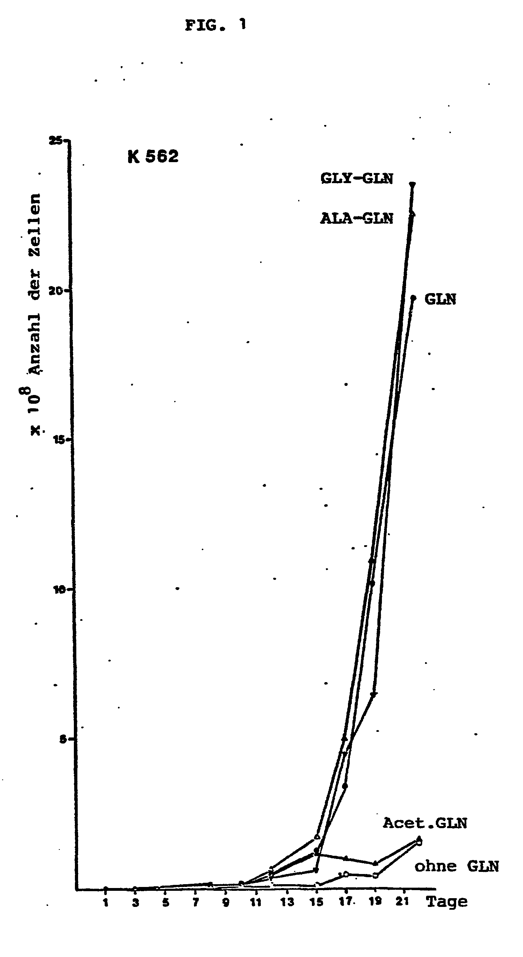

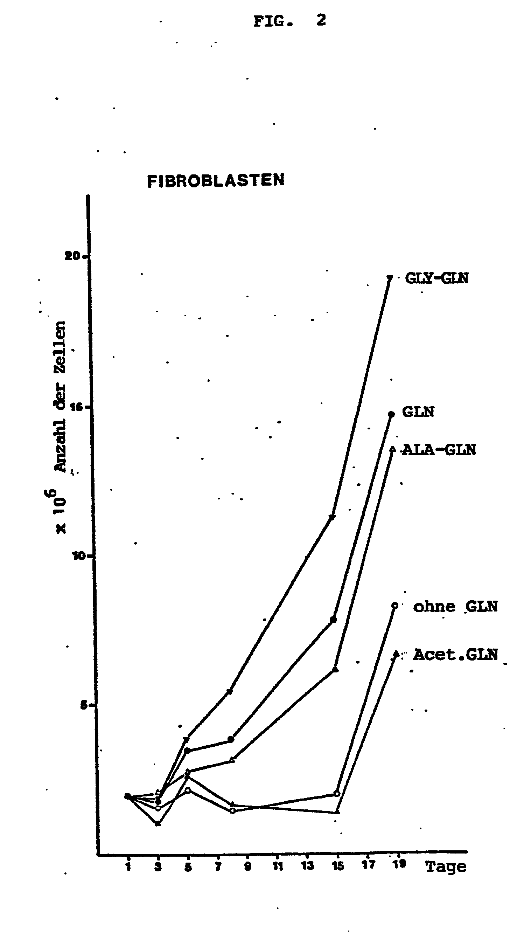

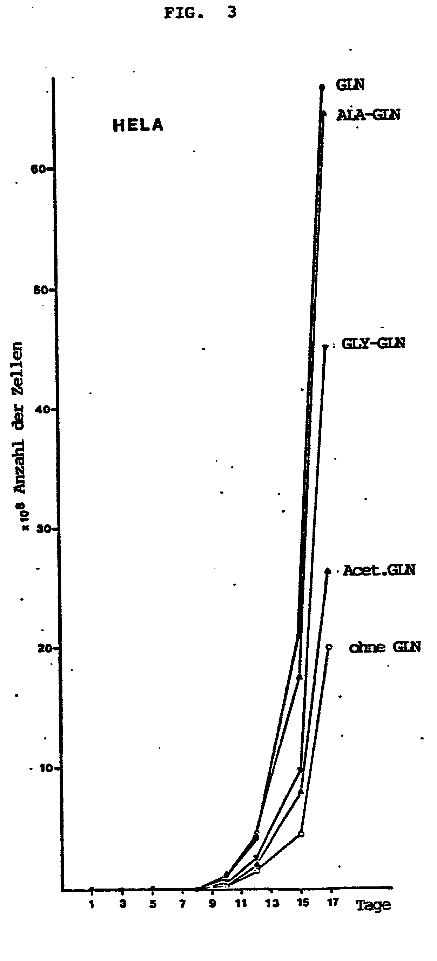

Bei den nachfolgend beschriebenen Tests mit Zellkulturen von drei verschiedenen Zellinien (K-562; Fibroblasten: Flow 4000; HELA 1L Passage) wurde untersucht, inwieweit die Dipeptide Alanyl-Glutamin oder Glycyl-Alanin das Glutamin als Wachstumsfaktor ersetzen können. Die Zellen wurden in glutaminfreiem RPMI 1640 (5% $CO_2$-Inkubator) gezüchtet. Ein Liter Medium enthielt 900 ml RPMI-1640, 100 ml fötales Kälberserum (über Nacht gegen PBS dialysiert) und 2 ml Gentamycinlösung. Dem Medium wurden 10 ml einer Lösung, die 200 mmol an Glutamin oder an ALA-GLN, GLY-GLN oder Acetylglutamin enthielt, zugesetzt (Sterilfiltration mit Acro Disc 200 µm Filter). Die K-562 Zellen wurden dreimal in der Woche auf 1/3 die HELA-Zellen dreimal auf 1/5 und die Fibroblasten zweimal auf 1/3 gespalten. Die Spaltung der HELA-Zellen und Fibroblasten erfolgte mittels Trypsin nach einmaliger Waschung mit PBS. Die Zellzahl wurde mit einem Sysmex-Microcell-Counter CC-10 ermittelt und nach jeder Spaltung hochgerechnet. Die Diagramme der Fig. 1 - 3 zeigen die Zellentwicklung mit und ohne Zugabe von Glutamin (GLN) und der jeweiligen Dipeptide bzw. des Acetylglutamins (Acet.GLN).

Wie hieraus ersichtlich, ist die Wachstumsgeschwindigkeit der einzelnen Zellinien bei Supplimentation des Mediums mit Glutamin oder den Dipeptiden: Alanyl-Glutamin und Glycyl-Glutamin annähernd gleich, während das Acetylglutamin eine wesentlich eingeschränkte Wachstumsgeschwindigkeit bewirkt.

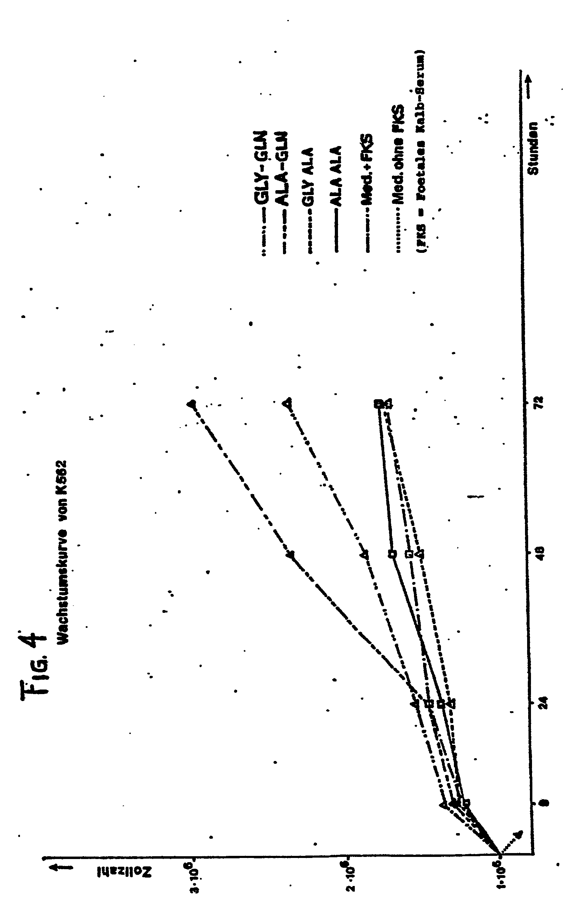

In weiteren Versuchen wurde das Wachstumsverhalten der Zellinie K-562 in Kulturmedien mit und ohne Zusatz der erfindungsgemäß zu verwendenden Peptide des Glutamins näher untersucht. Aus dem Diagramm der Fig. 4 ist ersichtlich, daß in glutaminfreiem Nährmedium weder ALA-ALA noch GLY-ALA zu einer Wachstumsstimulation führen, während bei Zusatz der Glutaminpeptide ALA-GLN und GLY-GLN die Zellzahl signifikant höher als im glutaminfreien Kulturmedium war, auch wenn dieses foetales Kalbserum (FKS)enthielt.

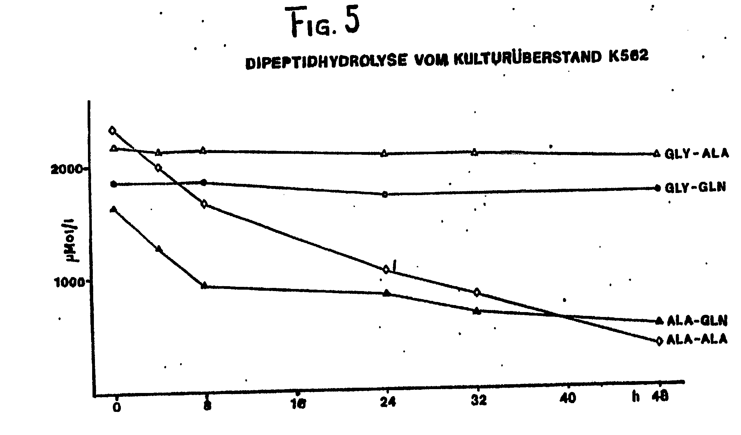

Untersucht wurde auch die Dipeptidhydrolyse im zellfreien, sterilfiltrierten Kulturüberstand. Wie aus Fig. 5 ersichtlich, werden die Dipeptide mit unterschiedlicher Geschwindigkeit von einem zellfreien, sterilfiltrierten K-562-Kulturüberstand hydrolysiert. Diese Untersuchung bestätigt die Befunde, die aus Fig. 4 erkennbar sind, nämlich, daß das ALA-GLN besser als GLY-GLN von der Zellinie K-562 gespalten wird.

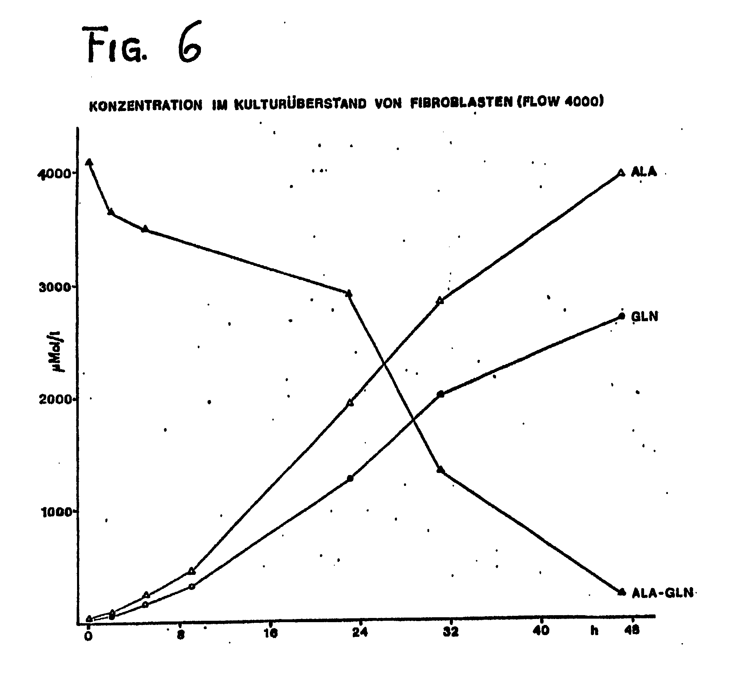

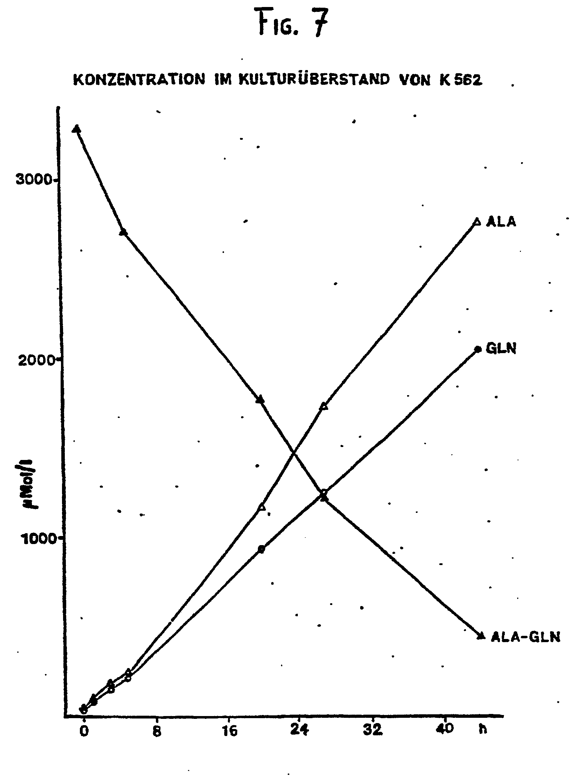

Die Spaltung von ALA-GLN durch wachsende Zellkulturen (K-562 und Fibroblasten: Flow 4000) ist den Fig. 6 und 7 zu entnehmen, die den Konzentrationsverlauf von ALA-GLN sowie den von Alanin und Glutamin während des Wachstums der zwei verschiedenen Zellinien zeigen. Hieraus ist ersichtlich, daß das ALA-GLN während des Wachstums kontinuierlich gespalten wird, wobei die Konzentrationen von Alanin und Glutamin zunehmen. Der geringere Konzentrationsanstieg von Glutamin im Verhältnis zu Alanin ist dadurch erklärbar, daß Glutamin während des Wachstums der Zellen stärker verstoffwechselt wird als das Alanin.

Diese Ergebnisse zeigen, daß Dipeptide von Zellkulturen unterschiedlich verwertet werden, wobei aber die Glutaminpeptide gut metabolisieren und zu einem signifikant besseren Zellwachstum führen.

## Patentansprüche

1. Verwendung von Di- und/oder Tripeptiden des Glutamins in Aminosäuren, Glucose und Mineralsalze enthaltenden Zellkulturmedien zur Züchtung isolierter Gewebszellen.

2. Verwendung der Dipeptide Glycyl-Glutamin und Alanyl-Glutamin in Zellkulturmedien nach Anspruch 1.

3. Verwendung der Di- und/oder Tripeptide des Glutamins nach Anspruch 1 oder 2 in Zellkulturmedien für die extrakorporale Insemination.

4. Verwendung der Di- und/oder Tripeptide des Glutamins nach Anspruch 1 - 3 in Zellkulturmedien für die Herstellung monoklonaler Antikörper.

## Claims

1. Use of di- and/or tripeptides of glutamine in cell culture media containing amino acids, glucose and mineral salts for cultivating isolated tissue cells.

2. Use of the dipeptides glycyl glutamine and alanyl glutamine in cell culture media according to claim 1.

3. Use of the di- and/or tripeptides of glutamine according to claim 1 or 2 in cell culture media for extracorporeal insemination.

4. Use of the di- and/or tripeptides of glutamine according to claims 1 to 3 in cell culture media for producing monoclonal antibodies.

## Revendications

1. Utilisation des di- et/ou tripeptides de la glutamine dans des milieux de culture de cellules contenant des acides aminés, du glucose et des sels minéraux, pour la culture de cellules de tissus isolées.

2. Utilisation des dipeptides glycyl-glutamine et alanyl-glutamine dans des milieux de culture de cellules selon la revendication 1.

3. Utilisation des di- et/ou tripeptides de la glutamine selon la revendication 1 ou 2, dans des milieux de culture de cellules pour l'insémination extracorporelle.

4. Utilisation des di- et/ou tripeptides de la glutamine selon les revendications 1 à 3, dans des milieux de culture de cellules pour la préparation d'anticorps monoclonaux.

EP 0 220 379 B1

FIG. 1

FIG. 2

FIBROBLASTEN

FIG. 3

FIG. 4    Wachstumskurve von K562

FIG. 5

DIPEPTIDHYDROLYSE VOM KULTURÜBERSTAND K562

EP 0 220 379 B1

# FIG. 6

KONZENTRATION IM KULTURÜBERSTAND VON FIBROBLASTEN (FLOW 4000)

EP 0 220 379 B1

# Fig. 7

### KONZENTRATION IM KULTURÜBERSTAND VON K 562